# EUROPEAN PATENT APPLICATION

(11) **EP 4 411 381 A1**
(43) Date of publication of application: **07.08.2024**
(21) Application number: 22876416.3
(22) Date of filing: 29.09.2022
(51) Int. Cl.: G01N 35/00

(54) **DATA ANALYSIS METHOD AND SERVER DEVICE**

(30) Priority: 01.10.2021 JP 2021162992
(71) Applicant: HITACHI HIGH-TECH CORPORATION, Tokyo 105-6409 (JP)
(72) Inventor: IMI Takuya, Tokyo 105-6409 (JP); TAKAHASHI Kenichi, Tokyo 105-6409 (JP); OOTAKI Tomotada, Tokyo 105-6409 (JP); SAGAE Nozomi, Tokyo 105-6409 (JP)
(74) Representative: Strehl Schübel-Hopf & Partner
(86) International application number: PCT/JP2022/036354
(87) International publication number: WO 2023/054550

(57) **Abstract**

An accurate alarm countermeasure against a malfunction can be rapidly executed when an alarm is generated while a user is using an automatic analyzer. The present disclosure is a data analysis method in which information regarding countermeasures of an alarm generated from a specific automatic analyzer is provided to the specific automatic analyzer using a computer, the data analysis method including: collecting alarm countermeasure reference information including information regarding various alarms and information regarding alarm countermeasures corresponding to the various alarms from a plurality of automatic analyzers; and analyzing the information regarding the alarm generated from the specific automatic analyzer, extracting an alarm countermeasure that is effective for releasing the alarm from the alarm countermeasure reference information, and transmitting the extracted alarm countermeasure to the specific automatic analyzer, in which the extraction of the alarm countermeasure from the alarm countermeasure reference information and the transmission of the extracted alarm countermeasure to the specific automatic analyzer includes narrowing down the alarm countermeasure reference information based on facility characteristic information including information regarding a use mode of the specific automatic analyzer that is acquired from the specific automatic analyzer and acquiring information regarding countermeasures suitable for the alarm that is generated from the specific automatic analyzer, rearranging the acquired information regarding the countermeasures suitable for the alarm based on a predetermined criterion, and providing the rearranged information regarding the countermeasures suitable for the alarm to the specific automatic analyzer via a communication line (refer to Fig. 4) .

## Description

### Technical Field

The present disclosure relates to a data analysis method and a server device.

### Background Art

In an automatic analyzer that executes biochemical and immunological component analysis of blood or urine, when an abnormality is detected during a device operation, an alarm is notified. This alarm is classified into two types including a system alarm and a data alarm. When an abnormality on a device such as a pressure sensor abnormality is detected, the system alarm is notified. When an abnormality of an analysis result such as a calibration abnormality is detected, the data alarm is notified. When the alarm is notified, in an automatic analyzer in the related art, an alarm name, an importance, a portion of generation, a description, and an alarm countermeasure are displayed on an alarm information screen. At this time, a plurality of alarm countermeasures are displayed, and are arranged in order set by a manufacturer. When all the displayed alarm countermeasures are randomly executed, most of the alarm countermeasures can be executed by only an operator. However, when one alarm countermeasure takes, for example, one hour, and the displayed alarm countermeasures are randomly executed, an unnecessary alarm correspondence time is taken. For example, in a case where an alarm is generated in an emergency hospital where only one automatic analyzer is provided, even when an urgent sample is received during alarm correspondence, there is a possibility that an accurate analysis result cannot be obtained. Therefore, a reduction in alarm correspondence time caused by a rapid alarm correspondence is required.

In order to make a rapid alarm correspondence, it is important to preferentially execute an alarm countermeasure having higher effectiveness than a plurality of alarm countermeasures, and experience of alarm countermeasures is important to determine the effectiveness of an alarm countermeasure. For example, a cause for a linearity abnormality that is a data alarm is water dripping from a cleaning mechanism or a variation in light intensity depending on the lifetime of a light source lamp, and the alarm is generated by various causes of generation. This way, when various portions of cause of generation can be considered for the same alarm, past experience or the like is required to narrow down a plurality of alarm countermeasures, and thus alarm countermeasure experience where past experience is accumulated is required. An experienced operator having a lot of alarm correspondence experience can determine an alarm countermeasure having high effectiveness based on past experiences or a device maintenance status. However, an operator having little alarm correspondence experience has little experience for determining an alarm countermeasure having high effectiveness, and thus cannot determine an alarm countermeasure having high effectiveness.

As described above, a countermeasure having high effectiveness is preferentially executed during generation of an alarm, and rapid release of the alarm is required. However, with the current automatic analyzer, the operator having little alarm correspondence experience cannot correspond to the alarm rapidly.

Under these circumstances, for example, PTL 1 describes that, by collecting alarm information and alarm countermeasures from devices all over the country and rearranging the collected alarm countermeasures in order from an alarm countermeasure having the most use cases, the effectiveness levels of the alarm countermeasures are compared such that an alarm countermeasure to be executed next and an alarm countermeasure that is considered to have the highest effectiveness are notified for each of the countermeasures to reduce the alarm correspondence time. The notified alarm countermeasures are rearranged in order from the alarm countermeasure that is mostly used based on the same alarm information. For example, when three countermeasures A to C are present against the generated alarm, assuming that the number of previous use cases of the countermeasure A is 3, the number of previous use cases of the countermeasure B is 5, and the number of previous use cases of the countermeasure C is 10, among the alarm countermeasures to be displayed, the countermeasure C having the most use cases is displayed first, the countermeasure B having the second most use cases is displayed second, and the countermeasure A having the third most use cases is finally displayed third. This way, PTL 1 discloses that a reduction in alarm correspondence time is attempted by rearranging the alarm countermeasures depending on the previous use statuses. In addition, when all the displayed alarm countermeasures are not effective for the alarm, the operator contacts a support center of an automatic analyzer manufacturer and receives a support for an additional countermeasure.

### Citation List

### Patent Literature

PTL 1: JP2009-300340A

### Summary of Invention

### Technical Problem

A general operator who operates the automatic analyzer is a clinical technologist who is professionally trained for a clinical examination. In addition, an operation method of the automatic analyzer changes depending on manufacturers or models. Therefore, the operator is trained by a manufacturer, a facility operator, or the like.

However, in order to select an alarm countermeasure having high effectiveness from a plurality of alarm countermeasures, the alarm countermeasure having high effectiveness is estimated based on not only knowledge such as an operation method but also past experience or a device maintenance status, and thus correspondence experience of an alarm (for warning a malfunction of the device) is important. In addition, for example, in a case where an alarm is generated in an emergency hospital where only one automatic analyzer is provided, even when an urgent sample is received during an alarm correspondence, there is a possibility that accurate analysis cannot be executed. Therefore, a reduction in alarm correspondence time is required to ensure the analysis time. Under these circumstances, a technique that allows even an operator having little alarm correspondence experience to select an alarm countermeasure having high effectiveness without randomly executing any countermeasure is required. That is, the development of a system that allows an operator having little alarm correspondence experience to select an alarm countermeasure having high effectiveness to reduce an alarm correspondence time is desired.

From this point of view, in PTL 1, alarm countermeasures are rearranged in order of effectiveness using only the number of use cases. However, the effectiveness of an alarm countermeasure is not determined depending on the number of use cases. For example, in a case where there is a facility where the device is used for 3 hours per day and a facility where the device is used for 24 hours per day, even when the device maintenance is executed in the same period, consumption speeds of components per day are different, and an alarm is more likely to be generated due to consumption of components in the device that is more frequently used. This way, depending on the device operational status of a user, an alarm that is likely to be generated changes, and the effectiveness of an alarm countermeasure changes correspondingly. Therefore, it is required to execute rearrangement of alarm countermeasures on which the device operational status is reflected.

In addition, in a device for which the maintenance has not been executed for longer than a recommended period, the occurrence frequency of an abnormality related to a maintenance item is considered to be increased. For example, regarding a device for which replacement of a tube is recommended every year, among a device where the replacement is executed half a year ago and a device where the replacement has not been executed for 3 years, the device where the replacement has not been executed for 3 years is more likely to generate an alarm due to cracking of the tube or the like. This way, the occurrence frequency of an abnormality changes depending on the maintenance status of the device, and the effectiveness of an alarm countermeasure changes correspondingly. Therefore, it is desired to execute rearrangement of alarm countermeasures on which the device maintenance status is reflected.

Under these circumstances, the present disclosure proposes a technique capable of rapidly executing an accurate alarm countermeasure against a malfunction when an alarm is generated while a user is using an automatic analyzer.

### Solution to Problem

In order to achieve the above-described object, the present disclosure provides a data analysis method in which information regarding countermeasures of an alarm generated from a specific automatic analyzer is provided to the specific automatic analyzer using a computer, the data analysis method including: collecting alarm countermeasure reference information including information regarding various alarms and information regarding alarm countermeasures corresponding to the various alarms from a plurality of automatic analyzers; and analyzing the information regarding the alarm generated from the specific automatic analyzer, extracting an alarm countermeasure that is effective for releasing the alarm from the alarm countermeasure reference information, and transmitting the extracted alarm countermeasure to the specific automatic analyzer, in which the extraction of the alarm countermeasure from the alarm countermeasure reference information and the transmission of the extracted alarm countermeasure to the specific automatic analyzer includes narrowing down the alarm countermeasure reference information based on facility characteristic information including information regarding a use mode of the specific automatic analyzer that is acquired from the specific automatic analyzer and acquiring information regarding countermeasures suitable for the alarm that is generated from the specific automatic analyzer, rearranging the acquired information regarding the countermeasures suitable for the alarm based on a predetermined criterion, and providing the rearranged information regarding the countermeasures suitable for the alarm to the specific automatic analyzer via a communication line.

Further characteristics related to the present disclosure will be clarified from the description of the present specification and the accompanying drawing. In addition, aspects of the present disclosure can be achieved and implemented by elements, a combination of multiple elements, the following detailed description and the scope of the appended claims. The description of the present specification is merely a typical example and does not limit the claims or application examples of the present disclosure by any means.

### Advantageous Effects of Invention

With the technique of the present disclosure, a user can accurately and rapidly take a countermeasure against a malfunction corresponding to an alarm generated from an automatic analyzer that is being used.

### Brief Description of Drawings

[Fig. 1] Fig. 1 is a diagram illustrating an overall configuration example of a multiple chemical automatic analyzer (hereinafter, simply referred to as "automatic analyzer") 100.
[Fig. 2] Fig. 2 is a diagram illustrating a configuration example of a screen (GUI) for setting a narrow-down condition for facility characteristics and a search range.
[Fig. 3] Fig. 3 is a diagram illustrating a graph (graph for a linearity abnormality) representing information regarding an absorbance corresponding to each of photometry points in a reaction of AST, and is a diagram illustrating a reaction process where water dripping occurs.
[Fig. 4] Fig. 4 is a diagram illustrating a schematic configuration example of an automatic analysis system (also referred to as "alarm analysis system") 10 where an abnormality alarm generated from the automatic analyzer 100 is analyzed by a support center (server device) 200 and a countermeasure of the alarm is provided to the automatic analyzer 100.
[Fig. 5] Fig. 5 is a flowchart illustrating an alarm countermeasure narrow-down process that is executed by the support center (server device) 200.
[Fig. 6] Fig. 6 is a diagram illustrating a table that stores maintenance items and a maintenance coefficient for each of the numbers of exceeding days.
[Fig. 7] Fig. 7 is a diagram illustrating an alarm countermeasure acquisition screen (large classification) related to abnormal data.
[Fig. 8] Fig. 8 is a diagram illustrating an alarm countermeasure acquisition screen (middle classification) related to abnormal data.
[Fig. 9] Fig. 9 is a diagram illustrating an alarm countermeasure acquisition screen (small classification) related to abnormal data.
[Fig. 10] Fig. 10 is a diagram illustrating a configuration example of a narrow-down condition setting screen according to a second embodiment.

### Description of Embodiments

An embodiment relates to an automatic analyzer that executes qualitative and quantitative analysis of a biological sample and particularly discloses a technique that can allow even an operator having little alarm correspondence experience to rapidly correspond to an alarm generated from one automatic analyzer such that a reduction in alarm correspondence time can be reduced.

Hereinafter, an embodiment of the present disclosure will be described with reference to the accompanying drawings. In the accompanying drawings, functionally the same elements may also be represented by the same reference numerals. The accompanying drawings illustrate specific embodiments and implementations based on the principle of the present disclosure. These drawings are examples for easy understanding of the present disclosure and are not used to limit the present invention.

In the present embodiment, the present disclosure is described in detail sufficient for a person skilled in the art to implement the present disclosure, but other embodiments and configurations can also be adopted. It should be understood that changes of configurations and structures and replacement of various elements can be made within a range not departing from the scope and concepts of the technical idea of the present disclosure. Accordingly, the following description should not be interpreted as being limited to the present disclosure.

Further, as described below, an embodiment of the present disclosure may be implemented by software operating on a general-purpose computer, or may be implemented by dedicated hardware or a combination of software and hardware.

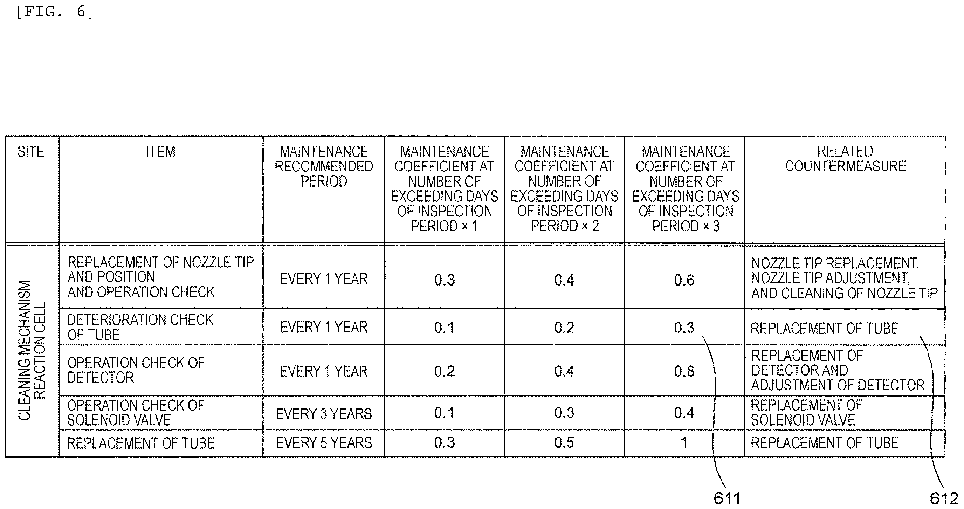

In the following description, each of information of the present disclosure will be described in the form of "table" (for example, refer to Fig. 6). The pieces of information do not need to be represented by a data structure using a table, and may be represented by a data structure of a list, a DB, a queue, or the like or other data structures. In order to show that various pieces of information do not depend on a data structure, "table", "list", "DB", "queue", or the like will also be simply referred to as "information".

In order to describe the content of each of information, the expressions "identification information", "identifier", "name", "ID", and the like can be used and can be replaced with each other.

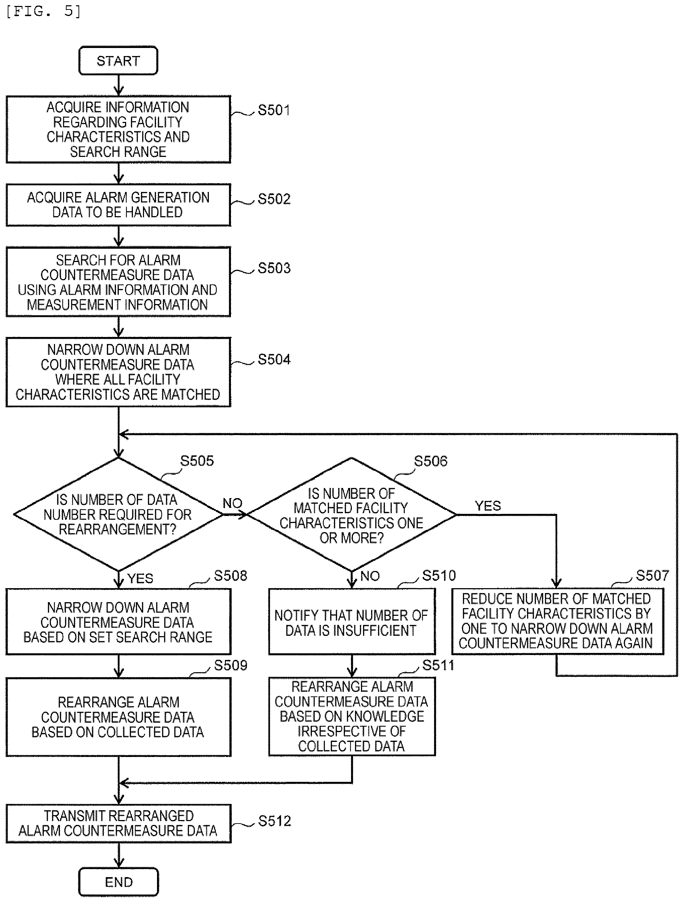

Hereinafter, each of processes (for example, each of steps in Fig. 5) in an embodiment of the present disclosure will be described by using "processing unit (corresponding to a processor)" of a support center (server device) as a subject (operation subject). However, a program corresponding to Fig. 5 may be an operation subject. In addition, some or all of the programs may be implemented by dedicated hardware or may be modularized. Various programs may be distributed and installed in a plurality of computers through a program distribution server or a storage medium.

### <Summary of Embodiment>

(i) Examples of causes for generating an alarm in the automatic analyzer include causes derived from a device, a sample, a reagent, and an operator. When an alarm is generated, it is difficult for an operator having little experience to estimate a cause for the alarm and to preferentially execute an alarm countermeasure having high effectiveness.
   In the present embodiment, in a system including a plurality of automatic analyzers (automatic analyzers of a plurality of users) that are connected to a support center (server device) via a dedicated line (network), alarm information is stored in a storage unit (for example, a storage device) of the support center from devices all over the country. When an alarm is generated from the automatic analyzer, data is narrowed down using the stored data, and countermeasures notified to an operator are rearranged in order from a countermeasure having the highest effectiveness. Data regarding the rearranged alarm countermeasures is transmitted to a target automatic analyzer via a network, and is displayed on a display screen of the automatic analyzer. The operator (user) can preferentially execute the alarm countermeasure having high effectiveness by simply executing the rearranged alarm countermeasures in order from the top, and a reduction in alarm correspondence time can be realized.
(ii) The summary of a method of collecting alarm countermeasure information generated in the device will be described (the more details refer to each of embodiments). The collected alarm countermeasure information relates to an alarm that is actually generated in the automatic analyzer. Examples of the information collected from the automatic analyzer include information (analysis date and time information) regarding a date on which analysis is executed, information regarding a reagent with which an alarm is generated, alarm identification information, maintenance status information, and alarm countermeasure information.

As the analysis date, a date stored in the device is used. As the information regarding a reagent with which an alarm is generated, a dedicated reagent code of data where an alarm is generated is read and collected. The dedicated reagent is a reagent that is sold based on a contract regarding a business alliance between a device manufacturer and a reagent manufacturer. The dedicated reagent is a reagent that is registered for allowing the device manufacturer and the reagent manufacturer that form the alliance to check the suitability of parameters and implementing a total service including performance guarantee to a customer. In addition, a code including information regarding a company that sells the reagent, an item, a lot, a prescription, and the like is allocated to the dedicated reagent, and is called the dedicated reagent code. By collecting the dedicated reagent code, the reagent can be identified. This information is acquired by a barcode reading unit reading a barcode of the reagent, and thus does not need to be input by the operator.

The alarm identification information is information for notifying what alarm is generated when the alarm is generated, and examples thereof include an alarm code, an alarm type, and an alarm number for identifying the alarm. By collecting the alarm identification information, the generated alarm can be identified. This information is included in the device, and thus does not need to be input by the operator.

The maintenance status information is information for identifying whether or not maintenance is executed within a maintenance period recommended by a manufacturer, and includes a date on which maintenance is executed, a maintenance recommended period, and the like. By collecting the maintenance status information, the rearrangement of alarm countermeasures on which the maintenance status is reflected can be executed. The date on which the maintenance is executed needs to be input, for example, after the maintenance. The maintenance date can be input from the automatic analyzer, and thus can be input from anybody. The alarm countermeasure information is information regarding what alarm is generated from any location of the device, an alarm countermeasure with which the alarm is released, and the like.

(iii) The alarm countermeasure information is information that is not collected from the device in the related art. Therefore, the method of collecting the alarm countermeasure information according to the present embodiment will be described below. For example, the device from which an alarm is generated takes a countermeasure against the alarm. Next, while seeing a screen of the automatic analyzer, the alarm countermeasure information such as a portion of generation, a cause of generation, or a countermeasure of the alarm is input. At this time, an input method is a selection expression using a selectable drawing, a pull-down, or the like and is basically only for selection. Therefore, an input that requires little time and effort and has high reproducibility is possible. In addition, in the input through the selection expression, options are formalized. Therefore, time and effort for a change into a format for data processing is not necessary, and the data processing is also easy.

This way, by collecting the alarm countermeasure information obtained through the input of the operator (user) and the dedicated reagent code and the alarm identification information stored in the automatic analyzer together, a computer (server device) of the support center can collect, from each of automatic analyzers, information regarding an alarm that is generated for each of reagents and an alarm countermeasure with which the alarm can be released. The alarm countermeasure can be directly input from the automatic analyzer, and thus can be input by anybody such as the operator, the manufacturer of the automatic analyzer, or the reagent manufacturer. The pieces of information are collected (accumulated) in a storage unit (for example, a storage device) of the support center using a dedicated line or the like.

(iv) In the present embodiment, the alarm countermeasure information is narrowed down based on the collected data. Hereinafter, an important narrow-down condition will be described. The narrow-down condition can be set from each of the automatic analyzers. The setting can be input by anybody such as the operator, the manufacturer of the automatic analyzer, or the reagent manufacturer. Once the setting is input, the setting does not need to be input again unless the setting is changed. Examples of the narrow-down condition include facility characteristics and a search range. Advantageous effects obtained by using the facility characteristics and search range for the narrow-down are as follows. That is, the facility characteristics are information to which a facility environment, a usage environment, and the like (a use mode of the device) are input for each of facilities. By executing the narrow-down using the facility characteristics, an abnormality that is frequently generated in a similar facility can be reflected on the rearrangement of alarm countermeasures. Examples of an item that is desired to be checked for determining whether or not abnormality is likely to occur due to maintenance failure include a usage time period and the number of days of use in one week. In addition, for example, when the device is used for 24 hours, a period of time for executing maintenance cannot be secured, and a problem is likely to occur due to maintenance failure. Therefore, in a facility where the device is used for 24 hours, an alarm generated by maintenance failure is more frequently narrowed down as compared to a facility where the device is not used too much.

As the search range, the number of recent data and a search period can be selected. By selecting the number of recent data, a recent trend of alarm countermeasures can be reflected. By executing the narrow-down using the search period, a trend in the selected data can be reflected on the rearrangement of alarm countermeasures. For example, a reaction between a reagent and a sample changes depending on an ambient temperature. Therefore, the use temperature is determined in the specification. In an area where the use temperature exceeds the specification depending on periods, in a period where analysis is executed at a temperature out of specification, a data alarm or the like is more likely to be generated due to the influence of the temperature, as compared to a period where analysis is executed at a temperature within specification. In this facility, by executing the rearrangement for each of acquisition periods (periods), the narrow-down on which the tendency of alarms for each of periods is reflected can be executed.

The narrow-down process of the alarm countermeasure information using the narrow-down condition is executed by the processing unit of the support center (server device). Therefore, data that is required to be narrowed down is transmitted from the automatic analyzer to the support center.

The data transmitted from the automatic analyzer to the support center will be described. For example, in PTL 1, information transmitted from the automatic analyzer when an alarm is generated includes alarm identification information and automatic analyzer identification information including a device serial number and a facility number for identifying the automatic analyzer where the alarm is generated. On the other hand, in the present embodiment, in addition to the above-described information (the alarm identification information and the automatic analyzer identification information), the information transmitted from the automatic analyzer includes the dedicated reagent code for identifying a reagent where an alarm is generated, the narrow-down condition including the facility characteristics and the search range that is information for narrowing down data to be collected, and the maintenance status information including the date on which maintenance is executed and the maintenance recommended period. When an alarm is generated, the pieces of information are automatically transmitted from the automatic analyzer to the support center via a line (network). In the process of the support center, the alarm countermeasure information stored in the data storage unit (for example, a storage device) is narrowed down using the transmitted information.

Regarding the narrow-down process of the alarm countermeasure information, first, the processing unit of the support center searches for data of which the alarm identification information and the dedicated reagent code match with those of the automatic analyzer from the automatic analyzer (device that is operated by a user who wants to know an alarm countermeasure effective for a generated alarm) using all the previous alarm countermeasure information. By executing the search, the alarm identification information generated when the same alarm is generated for the same reagent is narrowed down. Next, the processing unit further narrows down the data hit by the search using the facility characteristics. By executing the narrow-down using the facility characteristics, the alarm countermeasure information generated in a facility having the same facility characteristics can be narrowed down. Next, the processing unit further narrows down the data using the search range. By executing the narrow-down using the search range, the alarm countermeasure information in the set period can be narrowed down.

After executing the above-described narrow-down process, the processing unit of the support center executes a rearrangement process of the alarm countermeasure data. The rearrangement process is executed, for example, based on the number of use cases or the maintenance status. Specifically, the rearrangement process is executed, for example, using the maintenance status information including the number of use cases of each of alarm countermeasures, the date on which maintenance is executed, and the maintenance recommended period. By scoring the number of use cases and the maintenance status and adding up the scores for each of alarm countermeasures, an alarm countermeasure having a high score can be set as an alarm countermeasure having high effectiveness, and the alarm countermeasures can be rearranged in order from the alarm countermeasure having the highest effectiveness. In this case, regarding distribution of scores, for example, 1 point may be determined for 1 use case of an alarm countermeasure. Regarding the maintenance status, when there is a maintenance item where the maintenance recommended period is exceeded, for example, 10 points may be added to an alarm countermeasure related to the maintenance item. In addition, in a device where maintenance has not been executed for a longer period of time, an alarm is more likely to be generated due to maintenance failure. Therefore, for example, as the period of time when maintenance has not been executed increases, the score to be added may increase. In addition, in all the devices where maintenance has not been executed for the same period, the possibility of an alarm varies depending on maintenance items. Therefore, the score to be added may change depending on maintenance items. The possibility of an alarm depending on maintenance items may reflect the degree to which an alarm countermeasure related to the maintenance item is changed based on the collected previous alarm countermeasure data by comparing the number of use cases of the alarm countermeasure in a device where maintenance is executed within the recommended period and the number of use cases of the alarm countermeasure in a device where maintenance has not been executed for longer than the recommended period to each other. The above-described score (effectiveness) obtained by adding up the score for the number of use cases and the score for the maintenance status for each of the alarm countermeasures is obtained to rearrange the alarm countermeasures in order from the alarm countermeasure having the highest effectiveness.

As described above, after an alarm is generated, the narrow-down and the rearrangement of alarm countermeasures are automatically executed, and the operator executes the rearranged alarm countermeasures in order from the top. As a result, the alarm countermeasure having the highest effectiveness is preferentially executed. Therefore, even the operator having little alarm correspondence experience can preferentially execute the alarm countermeasure having the highest effectiveness, and the alarm correspondence time can be reduced.

### (1) First Embodiment

### <Overall configuration example of Automatic Analyzer>

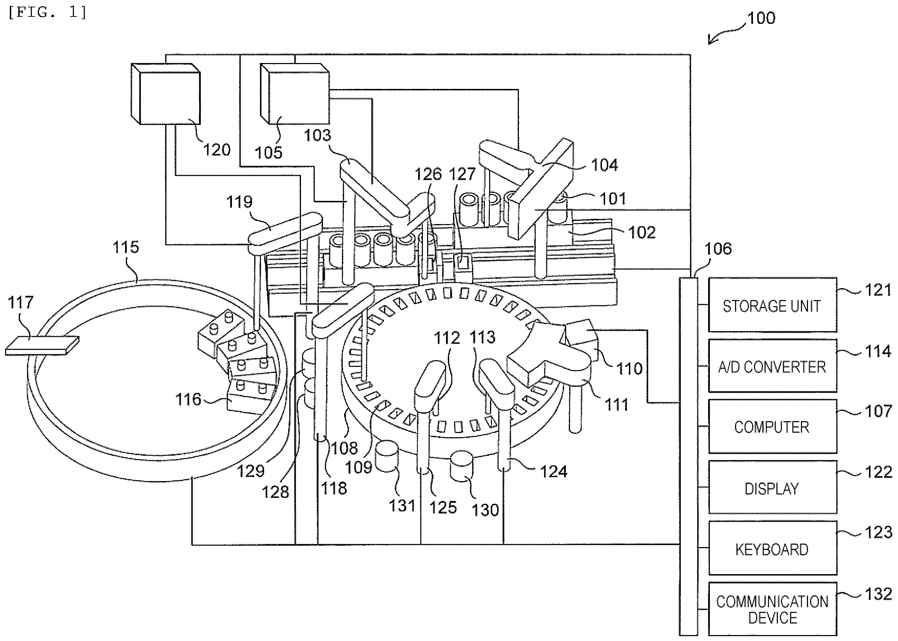

Fig. 1 is a diagram illustrating an overall configuration example of a multiple chemical automatic analyzer (hereinafter, simply referred to as "automatic analyzer") 100.

In the automatic analyzer 100 illustrated in Fig. 1, a plurality of sample containers 101 containing a sample are arranged in a sample rack 102. Aspiration/discharge nozzles of sample dispensing mechanisms 103 and 104 are connected to a syringe pump 105 for a sample. The operations of the pump 105 and the dispensing mechanisms 103 and 104 are controlled by a microcomputer 107 that controls the operation of each of the mechanism units and calculates measurement data via an interface 106. A plurality of reaction containers 109 are arranged on a reaction disk 108 to form a reaction line. A spectral photometer 110 is provided, and the reaction disk 108 is rotated and transported such that an optical beam from the spectral photometer 110 crosses an array of the reaction containers 109. The used reaction container 109 is cleaned by a cleaning mechanism 111 and is provided for reuse. Stirring rods 112 and 113 mix a sample added to the reaction container 109 and a reagent solution corresponding to an analysis item with each other. A measurement signal based on the reaction solution obtained by the spectral photometer 110 is converted from an analog signal into a digital signal by an A/D converter 114, and is input to the microcomputer 107.

In a reagent disk 115, multiple types of reagent bottles 116 corresponding to each of analysis items are provided along a circumference. That is, the disk 115 is a reagent bottle housing unit that is selectively rotatable. A barcode reading device 117 is provided on the disk 115. A reagent dispensing unit includes reagent probes 118 and 119 and a reagent syringe pump 120. The reagent probes 118 and 119 aspirate and hold predetermined amounts of the reagent solution in the reagent bottles 116 stopped at aspiration positions in the aspiration/discharge nozzles, rotate the aspiration/discharge nozzles on the reaction container array, and discharge the held reagent solution into the reaction containers 109 stopped at reagent reception positions. The reagent solution dispensed at this time is a solution of a type corresponding to an analysis item that is allocated to each of the reaction containers.

In each of the reagent bottles 116, a barcode is printed on a bottle outer wall. A numeral read from this barcode is called the dedicated reagent code. The dedicated reagent code includes information such as the size of the bottle, an expiry date of the reagent, a company determination code with which the reagent is sold, a sample item (prescription) determination code, or a reagent lot. The reagent information that is read from each of the reagent bottles 116 by the barcode reading device 117 is stored in a memory area corresponding to a storage unit 121 or the microcomputer 107. As the reagent bottle 116 is housed in the reagent disk 115, the reagent information is read by the barcode reading device 117. At this time, a signal representing a set position of each of the reagent bottles is output from a rotation angle detection unit provided in the reagent disk, and is input to the microcomputer 107 via the interface 106. The reagent information, the bottle set position, and the analysis item are stored in association with each other.

The operator (user) inputs various pieces of information using a display 122 and a keyboard 123. The measurement result of the analysis item can be checked using the display 122. In addition, parameters used in the analysis item are stored in the storage unit 121. The parameters are information such as a wavelength used in the spectral photometer, a sample amount, a calibration method, a standard solution concentration, the number of standard solutions, a check limit value of an analysis abnormality, a reagent type, a reagent dispensing order, a reagent bottle code, a reagent solution amount, a reagent dispensing amount, a maximum number of times of analysis, and a reagent manufacturing date.

The storage unit (for example, configured by a memory or a storage device) 121 stores not only various parameters but also operation conditions of each of the mechanism units of the automatic analyzer 100, analysis parameters of each of analysis items, a determination logic that manages each of reagent bottles, the maximum number of times of analysis read from the reagent bottle, an analysis result, and the like.

The analysis by the automatic analyzer 100 is executed in order of data processing such as sampling, reagent dispensing, stirring, photometry, cleaning of the reaction containers, or concentration conversion. Five sample containers 101 containing a sample are provided in one sample rack 102. The sample rack 102 is controlled by the microcomputer 107 via the interface 106. In addition, by arranging the sample containers 101 in the sample rack 102 in order of execution of analysis requests, the samples and the analysis items are associated with each other. Next, a predetermined sample container 101 is rotated and moved to a position below the sample probe according to the order of a sample to be analyzed, and a predetermined amount of the sample in the predetermined sample container 101 is dispensed into the reaction container 109 by the operation of the pump 105 for a sample linked to the sample probe. The sample probe is cleaned by sample probe cleaning mechanisms 126 and 127. The reaction container 109 into which the sample is dispensed moves up to a first reagent addition position. A predetermined amount of a reagent aspirated from the reagent bottle 116 is added to the moved reaction container 109 by the operation of the reagent pump 120 linked to the reagent probes. The used reagent probes are cleaned by reagent probe cleaning mechanisms 128 and 129, and the reaction containers 109 to which the reagent is added move up to positions of stirring mechanisms 124 and 125 and are stirred. After stirring, the stirring mechanisms clean the reaction containers 109 in stirring mechanism cleaning tanks 130 and 131. When first to third reagents are set in the reagent disk 115, the addition and stirring of the reagent are executed on the first to third reagents. The reaction container 109 of which the content is stirred passes through a light flux emitted from a light source, and the absorbance at this time is detected by the spectral photometer 110. The detected absorbance signal is input to the microcomputer 107 through the A/D converter 114 via the interface 106 to be converted into the concentration of the sample. The data converted into the concentration is stored in the storage unit 121 via the interface 106, and is displayed on the display 122. After the completion of the photometry, the reaction container 109 moves up to the position of the cleaning mechanism 111, is cleaned with a cleaning solution after discharging the content using a container cleaning pump, and is provided for the next analysis.

### <Setting of Narrow-Down Condition>

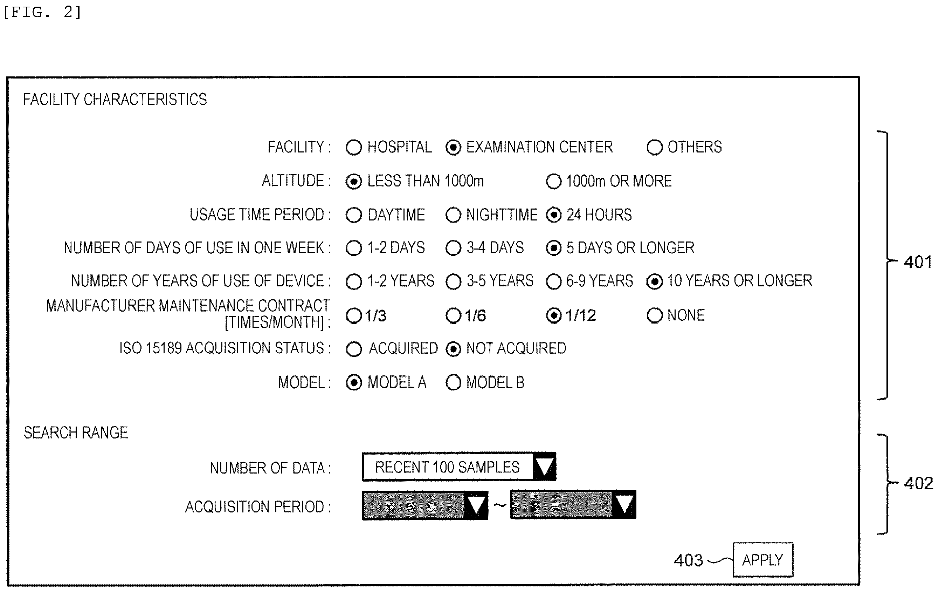

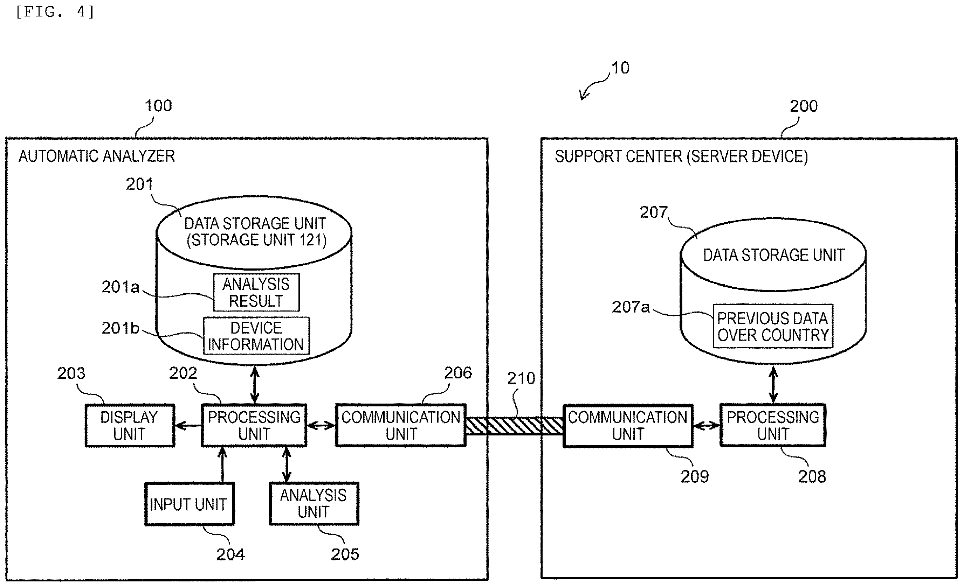

In the present embodiment (common in first and second embodiments), a support center (server device) 200 (refer to Fig. 4) uses the information collected from the plurality of automatic analyzers 100 to narrow down alarm countermeasures using the facility characteristics and the search range when an alarm is generated in the specific automatic analyzer 100, and rearranges the narrowed data. As a result, the alarm countermeasures are notified to the operator (user) of the specific automatic analyzer 100 in order from the alarm countermeasure having the highest effectiveness. To that end, the narrow-down condition is set for each of the automatic analyzers 100, and the setting content is stored in the storage unit 121 of the automatic analyzer 100. The narrow-down condition is used for reflecting a tendency in a similar facility or a trend in each of periods when the alarm countermeasure is notified. For example, as the narrow-down condition, facility characteristics (facility characteristic input field) 401 and a search range (search range input field) 402 are set by the user through a narrow-down condition setting screen (refer to Fig. 2: GUI) displayed by the display unit of the automatic analyzer 100. The setting of the narrow-down condition is directly executed from the automatic analyzer 100. Therefore, the setting can be input by anybody such as the operator, the device manufacturer, or the reagent manufacturer.

### (i) Regarding Facility Characteristics and Search Range Important for Narrow-Down

Fig. 2 is a diagram illustrating a configuration example of the screen (GUI) for setting the narrow-down condition for the facility characteristics and the search range. Here, the facility characteristics refer to a facility environment and a usage environment for each of facilities. In the present embodiment, by narrowing down the alarm countermeasures using the facility characteristics, an element such as an abnormality that is frequently generated in a similar facility can be reflected on the rearrangement of alarm countermeasures. For example, items of the facility characteristics include facilities, an altitude, a usage time period, the number of days of use in one week, the number of years of use of the device, a manufacturer maintenance contract status, an acquisition status of an ISO certification such as ISO 15189 or other international certifications, and a model, and these items are called facility characteristic items. By setting the facility characteristic items, the following effects can be obtained in each of the items.

For example, the setting item of the facility includes options such as a hospital, an examination center, and others. The operational status of the device varies depending on facilities. Therefore, the alarm countermeasure generation cause tendency by the variation in operational status can be reflected on the alarm countermeasures.

For example, the item of the altitude includes options such as 1000 m or more and less than 1000 m. As the altitude increases, the atmospheric pressure decreases. A difference in altitude affects, for example, the suction force of a vacuum pump using a difference in pressure. For example, in the cleaning mechanism 111, a vacuum pump for aspirating a detergent to clean the reaction container may be used. The aspirated detergent is diluted with water and is used as a cleaning solution for cleaning. At this time, in an area having a high air pressure, the force to aspirate the detergent is weaker than that in a region having a low air pressure. The detergent as the cleaning solution is diluted, and the detergency decreases to some extent. Of course, as long as the detergent is used within specification presented by the manufacturer, there is no effect on the analysis result. However, when another factor that decreases the detergency, for example, the clogging of a nozzle or a tube in the cleaning mechanism is also affected, an alarm is more likely to be generated due to insufficient cleaning in the area having a high air pressure. This way, the alarm countermeasure generation cause tendency by cleaning failure or the like can be reflected on the alarm countermeasures.

For example, the item of the usage time period includes options such as daytime, nighttime, and 24 hours. When the device operates for 24 hours, there is a possibility that the maintenance time cannot be ensured. Therefore, the alarm countermeasure generation cause tendency by maintenance failure or the like can be reflected on the alarm countermeasures. For example, the item of the number of days of use in one week options such as 1-2 days, 3-4 days, and 5 days or longer. The consumption speed of the device changes depending on the number of days. Therefore, the alarm countermeasure generation cause tendency by maintenance failure can be reflected on the alarm countermeasures. For example, the item of the number of years of use of the device includes options such as 1-2 years, 3-5 years, 6-9 years, and 10 years or longer. The malfunction occurrence rate changes depending on the number of years. Therefore, the alarm countermeasure generation cause tendency by device malfunction can be reflected on the alarm countermeasures. For example, the item of the manufacturer maintenance contract includes options such as 1 time/3 months, 1 time/6 months, 1 time/half year, and 1 time/year, and the quality of the device maintenance changes depending on whether there is maintenance contract. Therefore, the alarm countermeasure generation cause tendency by a failure that cannot be checked by normal inspection can be reflected on the alarm countermeasures.

For example, the acquisition status of the ISO certification such as ISO 15189 includes options such as acquired and not acquired. The acquisition of the ISO certification such as ISO 15189 represents that the state of the automatic analyzer 100 is good and the examination process is also executed according to the procedure. Accordingly, the alarm countermeasure generation cause tendency by regular maintenance, accuracy management, and the like can be reflected on the alarm countermeasures.

Examples of the model include a model A and a model B. For example, depending on the models of the automatic analyzer 100, some components are not mounted, and an alarm regarding these components is not generated. Therefore, the tendency of an alarm generated from the model where the component is used can be reflected on the alarm countermeasures.

For example, when the examination center, less than 1000 m, 24 hours, 5 days or longer, 10 years or longer, 1 time/year of the manufacturer maintenance contract, not acquired of the ISO certification such as ISO 15189, and the model A are selected in the facility characteristic items, the usage of the automatic analyzer 100 is high. However, the manufacturer maintenance period (frequency) is small at 1 time/year for the high usage. Therefore, the tendency of an alarm or the like that is relatively frequently generated due to deterioration of a component or the like is reflected on the alarm countermeasures to execute the narrow-down.

The data that is narrowed down using the facility characteristics can be further narrowed down using the information regarding the search range. The information regarding the search range includes "the number of recent data" and "the search period". For example, when the user selects "the number of recent data", a recent trend of alarm countermeasures can be reflected. In addition, when the user designates "the search period", the trend in the selected period can be reflected. By narrowing down the collected data in each of the search periods, the data collection on which the trend in the narrowed down period is reflected can be executed.

The operator (user) sets the facility characteristics and the search range and subsequently presses an apply button 403 in the same screen (GUI). As a result, the setting content can be stored in a data storage unit 201 (storage unit 121) of the automatic analyzer 100. The setting of the facility characteristics and the search range can be input by anybody, and the initial registration may be executed during device installation.

### <Example of Malfunction of Automatic Analyzer>

In the present embodiment, as an example of the malfunction (abnormality), a case where a crack is formed in a tube of the cleaning mechanism of the automatic analyzer 100 during analysis of a reagent item AST is assumed. When a crack is formed in the tube, air flows into the tube through a gap of the crack, and water is dripped from a tip of the cleaning nozzle due to the inflow of the air. The dripped water enters the reaction container such that the concentration of the content in the reaction container decreases during the analysis. At this time, a data alarm of a linearity abnormality may be generated. In this case, a countermeasure is taken against the alarm, and data collection after the countermeasure is executed.

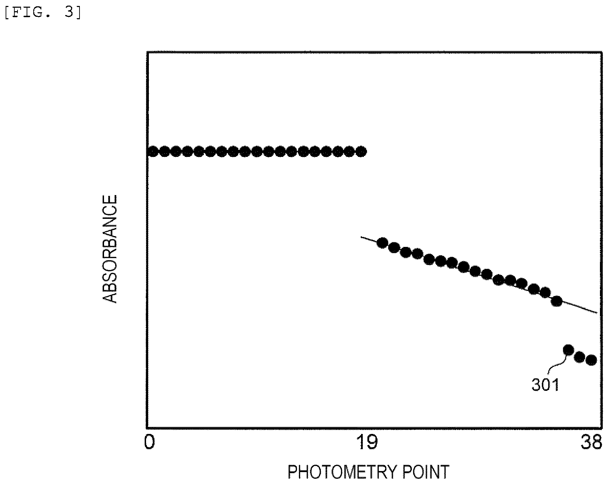

Fig. 3 is a diagram illustrating a graph (graph for a linearity abnormality) representing information regarding an absorbance corresponding to each of photometry points in a reaction of AST. For example, as illustrated Fig. 3, when an analysis item of a rate method such as the reaction process of the AST is normal, all the analysis results in the reaction process after secondary reagent addition show a linear reaction as in photometry points 20 to 35. In this analysis item, when analysis results that show a reaction deviating from the linear reaction occur as in photometry points 36 to 38, the linear abnormality alarm is notified.

### <System Configuration Example>

Fig. 4 is a diagram illustrating a schematic configuration example of an automatic analysis system (also referred to as alarm analysis system) 10 where an abnormality alarm generated from the automatic analyzer 100 is analyzed by the support center (server device) 200 and a countermeasure of the alarm is provided to the automatic analyzer 100. The automatic analysis system 10 includes at least one automatic analyzer 100, the support center (server device) 200, and a line (network) that connects the automatic analyzer 100 and the support center 200.

The automatic analyzer 100 includes various configurations illustrated in Fig. 1. The data storage unit 201 of Fig. 4 corresponds to the storage unit 121 of Fig. 1. The data storage unit 201 includes an analysis result storage area 201a and a device information storage area 201b. A processing unit 202 and an analysis unit 205 of Fig. 4 correspond to the computer 107 of Fig. 1. A display unit 203 of Fig. 4 corresponds to the display 122 of Fig. 1. An input unit 204 of Fig. 4 corresponds to the keyboard 123 of Fig. 1. A communication unit 206 of Fig. 4 corresponds to the communication device 132 of Fig. 1.

The support center (server device) 200 includes: a data storage unit 207 that is configured by a storage device or a memory and stores previous data 207a over the entire country (data regarding an abnormality alarm collected from each of the automatic analyzers 100 and an alarm countermeasure thereof); and a processing unit 208 that is configured by a computer (processor) and executes the narrow-down process, the rearrangement process, and the notification process.

In Fig. 4, first, the analysis unit 205 of the automatic analyzer 100 executes analysis. For example, when leakage occurs during the analysis, the reaction process of the item AST illustrated in Fig. 3 is obtained. As illustrated in Fig. 3, the analysis of the AST is normally executed until the photometry point 35. It can be seen that water dripping caused by the leakage occurs at the photometry points 36 to 301. When the dripped water enters the reaction container, the concentration of the sample or the reagent in the reaction container decreases due to the water with an absorbance of 0. Therefore, a decrease in absorbance occurs after the photometry points 36 to 301.

The result obtained by the analysis unit 205 of the automatic analyzer 100 is processed by the processing unit 202 on the automatic analyzer 100 side. As a result, whether or not the measurement result is normal or abnormal is determined. In the present analysis result, after the photometry points 36 to 301 where the absorbance decreases, the linearity check value (absorbance) for checking the linearity of the reaction exceeds a parameter set value of each of analysis items stored in the device, and the linearity of the reaction is lost. Therefore, the linear abnormality alarm is generated.

The linear abnormality alarm information is stored in the analysis result storage area 201a of the storage unit 201 of the automatic analyzer as a part of the analysis results. The storage unit 201 includes the analysis result storage area 201a where the analysis result is stored and the device information storage area 201b where the device information is stored. In the device information storage area 201b, device identification information, the dedicated reagent code, the analysis date, the narrow-down condition, and the maintenance status information are stored. The device identification information refers to information including a device serial number and a facility number for identifying the automatic analyzer where the alarm is generated, and the device identification information over the entire country is stored in a previous data storage area 207a over the entire country of the data storage unit 207 of the support center (server device) 200.

When the analysis result is stored in the data storage unit 201 (analysis result storage area 201a), the automatic analyzer 100 automatically collects the alarm countermeasure information regarding the linear abnormality alarm using the stored data. The flow of the alarm countermeasure information collection is as follows. First, at this time, the processing unit 202 reads not only the analysis result data that is stored in the analysis result storage area 201a and where the linear abnormality alarm is generated but also the device information 201b from the data storage unit 201 (storage unit 121) of the automatic analyzer 100. The processing unit 202 controls the communication unit 206 of the automatic analyzer 100 to transmit the read data to a communication unit 209 of the support center 200 via a line (network) 210. The processing unit 208 of the support center 200 searches data stored in the previous data storage area 207a over the entire country based on the alarm identification information and the dedicated reagent code in the data received by the communication unit 209. In the previous data storage area 207a over the entire country, the previously collected alarm correspondence information is stored. The target to be searched at this time is "all the data where the linear abnormality alarm is generated with the same reagent code". The processing unit 208 on the support center 200 side executes narrow-down process on the read (searched) previous data over the entire country based on the narrow-down condition set in Fig. 2. The details of the narrow-down process will be described below (refer to Fig. 5).

### <Details of Narrow-Down Process>

Fig. 5 is a flowchart illustrating an alarm countermeasure narrow-down process that is executed by the support center (server device) 200. An operation subject of each of steps is basically the processing unit (the processor or the computer) 208. A narrow-down process program corresponding to the flowchart of Fig. 5 may be the operation subject.

### (i) Step 501

The processing unit 208 acquires (receives) the information of the narrow-down condition regarding the facility characteristics and the search range from the automatic analyzer 100 where an alarm is generated. The setting of the facility characteristics and the search range is set in advance by the operator (user) in each of the automatic analyzers 100 through the GUI of Fig. 2. The setting content of the narrow-down condition is stored in the storage unit 121 (data storage unit 201) of each of the automatic analyzers 100. Therefore, the processing unit 202 of each of the automatic analyzers 100 acquires the information of the narrow-down condition (the facility characteristics and the search range) from the storage unit 121 when the alarm is generated, and transmits the acquired information to the support center (server device) 200 using the communication unit 206. The transmitted information of the narrow-down condition is received by the processing unit 208 of the support center 200. The support center 200 may receive the set information of the narrow-down condition from each of the automatic analyzers 100 in advance (for example, the time when the narrow-down condition is set in each of the automatic analyzers 100), and may be stored in the storage unit 207 in association with the identification information of the automatic analyzer 100.

### (ii) Step 502

The processing unit 208 acquires alarm generation data to be corresponded from the automatic analyzer 100 where the alarm is generated. The alarm generation data can include the measurement result data illustrated in Fig. 3, an alarm code, information regarding the type of the device, a device code (including device lot information), and a reagent code (including reagent lot information).

When an alarm is actually generated in the automatic analyzer 100, the alarm code, the measurement result, the reagent code, and the alarm generation date (analysis date) are stored in the analysis result storage area 201a. When the operator (user) of the automatic analyzer 100 inputs an instruction to inquire the support center 200 about the alarm countermeasure, the processing unit 202 puts together the alarm code, the measurement result, the reagent code, the alarm generation date, and the information regarding the device code or the type of the device to generate the alarm generation data, and transmits the alarm generation data to the support center 200 through the communication unit 206. As a result, the processing unit 208 of the support center 200 acquires the alarm generation data.

### (iii) Step 503

The processing unit 208 searches the previous data over the entire country (accumulation of the previous alarm countermeasure data) using the alarm information (for example, the alarm code) and the measurement information (for example, the reagent code or the device code) in the target alarm generation data, and acquires the alarm countermeasure data related to the same alarm information and the same measurement information.

### (iv) Step 504

The alarm countermeasure data obtained in Step 503 is data corresponding to all the facility characteristics and the entire search range (date). Accordingly, the search result of Step 503 also includes the alarm countermeasure data of the automatic analyzer 100 having different facility characteristics from those of the automatic analyzer 100 where the alarm is currently generated.

Therefore, the processing unit 208 narrows down the alarm countermeasure data where all the facility characteristics match with those of the target automatic analyzer 100 (where the alarm is currently generated) among the alarm countermeasure data obtained in Step 503. In the present embodiment, the settings (setting contents in Fig. 2) of the facility item are the hospital, less than 1000 m, 24 hours, 5 days or longer, 3-5 years, 1 time/year of the manufacturer maintenance contract, not acquired of the ISO certification such as ISO 15189, and the model A. Accordingly, the previous countermeasure data where the settings match with these setting contents are acquired.

### (v) Step 505

The processing unit 208 determines whether or not the number of the alarm countermeasure data obtained (narrowed) in Step 504 is collected by the number required for the data rearrangement (is more than or equal to a required number of data). Here, the number of data required for the data rearrangement can be the number of recent data or more, for example, when the alarm countermeasure data is narrowed down to the number of recent data using the search range 402, and can be 50 samples (example), for example, when the alarm countermeasure data is narrowed down using the period. In the present embodiment, the search range 402 is set to narrow down the alarm countermeasure data to recent 100 samples. Therefore, the number of 100 samples as the number of recent data is the required number of data.

When the alarm countermeasure data are present by the number of data required for the rearrangement (Yes in Step 505), the process proceeds to Step 508. When the alarm countermeasure data are insufficient for the number required for the rearrangement (No in Step 505), the process proceeds to Step 506.

### (vi) Step 506

The processing unit 208 determines whether or not the number of the facility characteristics of the narrowed alarm countermeasure data that match with the facility characteristics set in the automatic analyzer 100 where the alarm is generated is 1 or more, that is, whether or not the number of the matched facility characteristics is at least one. When the number of the matched facility characteristics is one or more (Yes in Step 506), the process proceeds to Step 507. When the matched facility characteristics are not present (No in Step 506), the process proceeds to Step 510.

### (vii) Step 507

The processing unit 208 reduces the number of the matched facility characteristics by one to narrow down the alarm countermeasure data again. In the present embodiment, for example, the number of the facility characteristic items is 8 including the facility, the sea level altitude, the usage time period, the number of days of use in one week, the number of years of use of the device, a manufacturer maintenance contract status, the acquisition status of the ISO certification such as ISO 15189, and the model. Therefore, the number of the matched facility characteristic items is reduced to 7 to narrow down the searched data again.

When the number of data insufficient even after executing the narrow-down again, the process of checking the number of the matched facility characteristic items (Step 506) → the process of reducing the number of the facility characteristics by one to narrow down the alarm countermeasure data (Step 507) is repeated until the number (for example, 100 samples) of data required for the narrow-down can be secured (check in Step 505).

### (viii) Step 508

When 100 samples or more required for the rearrangement can be secured as a result of the narrow-down using the facility characteristics (Yes in Step 505), the processing unit 208 narrows down the alarm countermeasure data using the search range. For example, when the setting of the search range 402 is recent 100 samples, the processing unit 208 can further narrow down the alarm countermeasure data (100 samples or more) that is narrowed down using the facility characteristics to recent 100 samples.

### (ix) Step 509

The processing unit 208 rearranges the alarm countermeasure data that is narrowed down using the facility characteristics and the search range based on the effectiveness determination criterion. The details of the rearrangement will be described below.

### (x) Step 510

The processing unit 208 notifies the automatic analyzer 100 via the communication unit 209 that the number of the alarm countermeasure data is insufficient for presenting the alarm countermeasures to the automatic analyzer 100 where the alarm is currently generated.

### (xi) Step 511

The processing unit 208 rearranges the alarm countermeasures based on experience irrespective of the collected alarm countermeasure data. As the alarm countermeasures based on the experience, for example, general alarm countermeasures corresponding to the type of the alarm code or alarm countermeasures determined by the manufacturer can be used and can be stored in the data storage unit 207 in advance. In addition, the predetermined alarm countermeasures are also rearranged in order from the alarm countermeasure having the highest effectiveness for various malfunctions/abnormalities (each of the alarm codes) (in order determined by the manufacturer side).

### (xii) Step 512

The processing unit 208 transmits the rearranged alarm countermeasure data to the target automatic analyzer 100 using the communication unit 209. The automatic analyzer 100 causes the display 122 (display unit 203) to display the received alarm countermeasure data in the rearranged order.

### (xiii) Others

The narrow-down process and the rearrangement process described above may be automatically executed by the support center (server device) 200, or may be automatically executed by the automatic analyzer 100 after acquiring the previous data.

### <Details of Rearrangement Process>

The rearrangement of the alarm countermeasure data is executed based on the effectiveness determination criterion. Here, the effectiveness determination criterion is, for example, a criterion in consideration of the number of use cases or the maintenance status. For example, the number of use cases of each of the alarm countermeasures in the narrowed data or the maintenance status information including the date on which maintenance is executed and the maintenance recommended period can be used. Here, for example, a method of scoring the number of use cases and the maintenance status and adding up the scores for each of alarm countermeasures such that an alarm countermeasure having a high score is set as an alarm countermeasure having high effectiveness to rearrange the alarm countermeasures in order from the alarm countermeasure having the highest effectiveness will be described. Hereinafter, the scoring method will be described.

### (i) Scoring of Number of Use Cases

First, the method of scoring the number of use cases will be described. The number of use cases is a value indicating the number of times each of the countermeasures in the narrowed alarm countermeasure data is used. The number of use cases is counted by the processing unit 208 of the support center (server device) 200. Regarding the scoring method, for example, 1 point is allocated to one use case of the alarm countermeasure.

### (ii) Scoring of Maintenance Status

The information regarding the maintenance status can be scored by multiplying the number of the narrowed alarm countermeasure data and the maintenance coefficient for each of the maintenance items. The maintenance coefficient is a coefficient representing the number of days elapsed from the maintenance recommend date and the alarm influence for each of the maintenance items, and can be stored in the support center in a form illustrated in Fig. 6 (table that stores the maintenance items and the maintenance coefficient for each of the number of exceeding days). The maintenance coefficient is, for example, a value obtained by acquiring a ratio between the number of use cases in the device where maintenance is executed within the maintenance recommended period and the number of use cases in a device where maintenance has not been executed for longer than the recommended period from the previous alarm countermeasure information.

As described above, by scoring the number of use cases and the maintenance status and adding up the scores for each of alarm countermeasures, an alarm countermeasure having a high score is set as an alarm countermeasure having high effectiveness, and the alarm countermeasures are rearranged in order from the alarm countermeasure having the highest effectiveness.

### (iii) Specific example of Scoring

Here, an example (case where the linear abnormality alarm is generated) of scoring based on the number of use cases and the maintenance status will be described. Examples of the alarm countermeasure when the linear abnormality alarm is generated include replacement of a solenoid valve (countermeasure A), replacement of a tube (countermeasure B), and replacement of a light source lamp (countermeasure C). In addition, in this example, 100 cases of previous data that are narrowed down using the search range 402 can be used. At this time, assuming that the number of use cases of the alarm countermeasure A is 20, the number of use cases of the alarm countermeasure B is 30, and the number of use cases of the alarm countermeasure C is 50, the alarm countermeasures are rearranged as follows.

When 1 point is allocated to one use case, the scoring by the number of use cases is as follows. The numbers of use cases are 20 in the countermeasure A, 30 in the countermeasure B, and 50 in the countermeasure C, and the scores are 20 points in the countermeasure A, 30 points in the countermeasure B, and 50 points in the countermeasure C.

When the maintenance status is scored by multiplying the maintenance coefficient by the number of the narrowed data, the scoring by the maintenance status is executed in the following flow. In the automatic analyzer 100 according to the present embodiment, assuming that "deterioration check of tube" that is one maintenance item has not been executed for 3 years, the maintenance coefficient is 0.3 in Fig. 6 (reference number 611). In addition, the number of data narrowed down by the setting of the search range 402 is 100. When 0.3 of the maintenance coefficient and 100 of the number of the narrowed data are multiplied by each other, the score of the maintenance status is 30 points. This score is added to only the alarm countermeasure related to maintenance item described in Fig. 6 without being added to all the alarm countermeasures. The alarm countermeasure related to "the deterioration check of the tube" where maintenance has not been executed is only "tube replacement" (countermeasure B) (reference number 612). Therefore, the scores by the maintenance status of the alarm countermeasures to be added in the present embodiment are 0 points in the countermeasure A, 30 points in the countermeasure B, and 0 points in the countermeasure C, respectively. When the number of items where maintenance has not been executed is two or more, the score of each of the items is calculated in the above-described flow and is added to the alarm countermeasures related to each of the items.

When the score of the number of use cases and the score of the maintenance status are added up for each of the countermeasures, the score of the countermeasure A is 20 points obtained by adding up 20 points of the number of use cases and 0 points of the maintenance status, the score of the countermeasure B is 60 points obtained by adding up 30 points of the number of use cases and 30 points of the maintenance status, and the score of the countermeasure C is 50 points obtained by adding up 50 points of the number of use cases and 0 points of the maintenance status. Therefore, the countermeasures in the example are rearranged in order of the score (effectiveness) that is the display order such that the countermeasure B having 60 points is displayed on the top, the countermeasure C having 50 points is displayed on the next line, and the countermeasure A having 20 points is displayed on the bottom. Accordingly, in the example, it can be seen that the alarm countermeasure having the highest effectiveness is the replacement of the tube.

As described above, the processing unit 208 of the support center 200 transmits the data where the alarm countermeasures are narrowed down and rearranged to the target automatic analyzer 100 via the communication unit 209 and the line 210. The target automatic analyzer (in the example, the automatic analyzer where the linearity alarm is generated) 100 is specified based on the automatic analyzer identification information including the device serial number and the facility number.

The list of the rearranged alarm countermeasure information is displayed in rearranged order on an analysis result screen of the automatic analyzer 100. The operator (user) checks the portion of cause of the alarm countermeasure that is displayed on the top and takes the alarm countermeasure. As a result, the alarm countermeasure having the highest effectiveness can be preferentially executed. In the example, "replacement of tube" (countermeasure B) is the most preferential alarm countermeasure. Therefore, the user checks a portion of generation (a system water discharge port of the cleaning mechanism) that is described together with the alarm countermeasure, and checks the cause (liquid dripping) that is described together with the alarm countermeasure. When liquid dripping occurs, "replacement of tube" is requested for the service. On the other hand, when liquid dripping does not occur, "replacement of light source lamp" having the second highest effectiveness is executed. This way, by preferentially executing the countermeasure having the highest effectiveness based on the rearranged alarm countermeasure information, even the operator having little alarm countermeasure experience can rapidly take a countermeasure, and the alarm correspondence time can be reduced.

### <Process of Collecting Alarm Countermeasure Information>

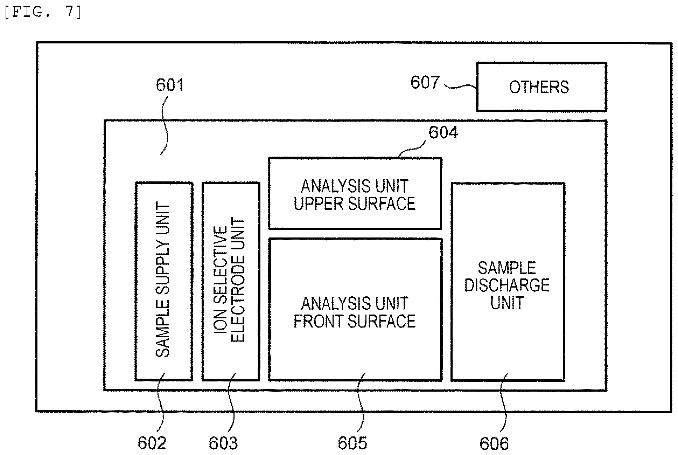

After taking the alarm countermeasure to release the alarm, the alarm countermeasure information is collected by the support center 200. When the alarm countermeasure information is collected by the support center 200, the operator (user) of the automatic analyzer 100 selects an alarm countermeasure report button on the analysis result screen (not illustrated) of the analysis result after taking the alarm countermeasure, and inputs necessary information using the countermeasure acquisition screen (refer to Figs. 7 to 9). In the present embodiment, the alarm is released by "replacement of tube" having the highest effectiveness, and the data collection is executed.

The data to be collected is the alarm identification information, the dedicated reagent code, the alarm countermeasure information, the maintenance status information, and the analysis date. The pieces of information are associated with each other and are collected by (transmitted to) the support center 200. The alarm identification information, the dedicated reagent code, and the analysis date are information that do not need to be input and are stored in the device, but the alarm countermeasure information and the maintenance status need to be input. This input can be executed by anybody.

Hereinafter, a method of inputting the alarm countermeasure information and the maintenance status will be described.

### (i) Input of Alarm Countermeasure Information

The alarm countermeasure information can be input by selecting necessary features according to the input screen. For example, when the alarm countermeasure information (portion of generation, cause of generation, and countermeasure) of the present embodiment are "system water discharge port of cleaning mechanism, cracking in tube, and replacement of tube", and this content is input in the following flow. First, the user selects a portion of a cause of abnormality in the automatic analyzer overall configuration (displayed on the input screen) illustrated in Fig. 7 (the diagram illustrating the countermeasure acquisition screen (large classification) related to the abnormal data). The automatic analyzer overall configuration includes a frame 601 representing a device front configuration and a frame 607 representing other abnormality cause areas. The device front configuration 601 represents the front surface of the devices and includes, as internal options, a frame 602 representing a sample supply unit, a frame 603 representing an ion selective electrode unit, a frame (analysis unit upper surface frame) 604 representing an analysis unit upper surface, a frame (analysis unit front surface frame) 605 representing an analysis unit front surface, a frame (sample discharge unit frame) 606 representing a sample discharge unit.

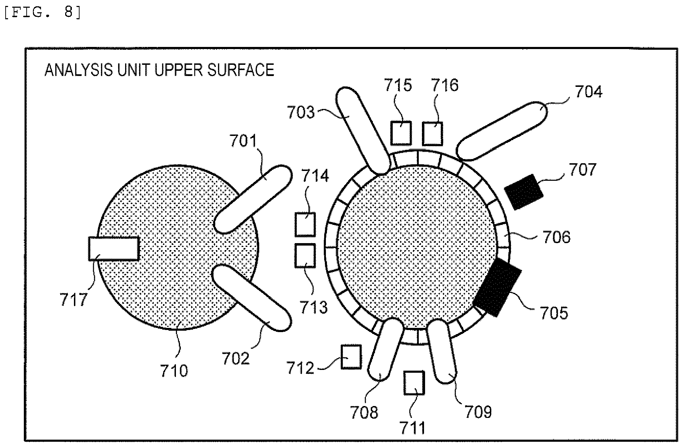

It is assumed that the present portion of cause is the system water discharge port of the cleaning mechanism. Therefore, the operator (user) selects the frame 604 representing the analysis unit upper surface where the cleaning mechanism is present. When the frame 604 representing the analysis unit upper surface is selected, the countermeasure acquisition screen (Fig. 8) of the drawing of the analysis unit upper surface is displayed on the screen.

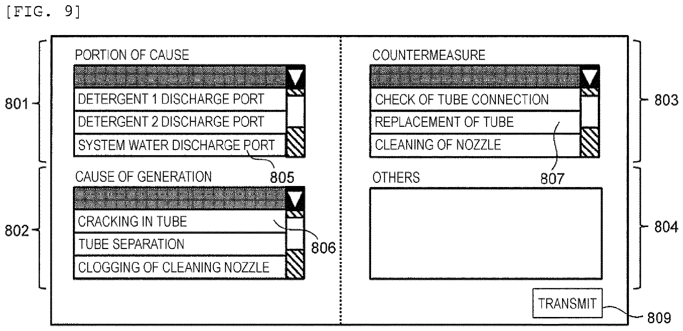

Fig. 8 is a diagram illustrating a countermeasure acquisition screen (middle classification) related to abnormal data (diagram illustrating the detailed configuration of the analysis unit upper surface). The analysis unit upper surface includes reagent probes 701 and 702, sample probes 703 and 704, a cleaning mechanism 705, a reaction container 706, a spectral photometer 707, stirring mechanisms 708 and 709, a reagent disk 710, cleaning tanks 711 to 716, and a barcode reading unit 717. The operator (user) selects a component where an abnormality occurs from these components. It is assumed that the present portion of cause is the system water discharge port of the cleaning mechanism. Therefore, the user selects the cleaning mechanism 705. When the cleaning mechanism 705 is selected, Fig. 9 is displayed.

Fig. 9 is a diagram illustrating an input screen configuration example of the portion of cause, the cause of generation, and the countermeasure (diagram illustrating a countermeasure acquisition screen (small classification) related to abnormal data). On the input screen illustrated in Fig. 9, locations for selecting a specific portion of cause (abnormality occurrence portion selection field) 801, a cause of generation (alarm generation cause selection field) 802, and a countermeasure (countermeasure selection field) 803 for making data normal are provided. At this time, it is assumed that a crack is formed in the tube of the system water discharge port and water is dripped from the system water discharge port due to the effect of the crack. The user selects "system water discharge port 805" as the portion of cause 801, and selects "cracking in tube 806" as the cause of generation 802. In addition, the malfunction is resolved by replacing the tube. Therefore, the user selects "replacement of tube 807" as the countermeasure 803.

When there are no options in the fields of the portion of cause 801, the cause of generation 802, and the countermeasure 803, the user fills the content in others field 804. The content filled in the others field 804 is transmitted to the support center 200. Referring to the transmitted content, for example, the service man or the like adds selection options to the portion of cause 801, the cause of generation 802, and the countermeasure 803. When a plurality of countermeasures are simultaneously taken, a plurality of items may be selected.

### (ii) Collection of Maintenance Status Information

The maintenance status information is collected by inputting the maintenance status information on the screen as in the alarm countermeasure information. The maintenance status information includes, for example, the maintenance recommended period and the date on which maintenance is executed. The maintenance recommended period is a period that is determined by the manufacturer and thus is stored in the support center. The information that is actually collected is the information regarding the date on which maintenance is executed, and this information is input. The maintenance status information is input from the automatic analyzer 100, and thus can be input from anybody. Regarding the input of the maintenance status information, only the date (A.D.) on which maintenance is executed is input for each of the maintenance items. This input is executed after every maintenance.

The data to be collected by the support center 200 is temporarily stored in the automatic analyzer 100. The transmission process to the support center 200 is started by the user finally pressing a transmit button 809. For example, the communication unit 206 of the automatic analyzer 100 transmits the alarm identification information, the dedicated reagent code, the alarm countermeasure information, and the maintenance status information to the support center 200 via the line (network) 210. The transmitted data is stored in the data storage unit 207 of the support center 200 and is subsequently used as the previous data for the narrow-down.

### (2) Second Embodiment

The second embodiment relates to a narrow-down process by setting priorities to the facility characteristic items. The other processes and devices and system configurations are the same as those of the first embodiment. Hereinafter, only differences from those of the first embodiment will be described.

### (1) Regarding Narrow-Down using Facility Characteristics

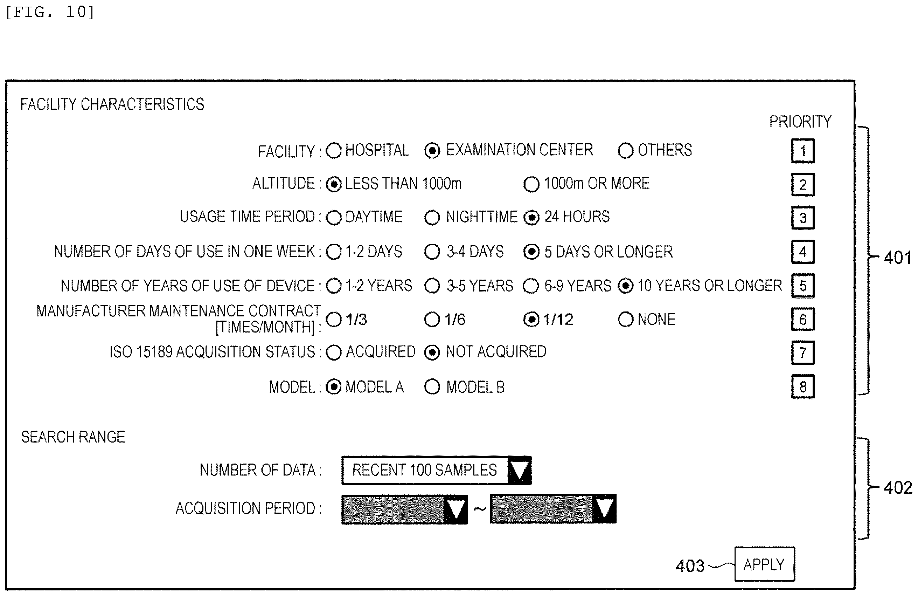

In the first embodiment, when the number of data is less than the number of recent data during the narrow-down using the facility characteristics, "the number of items" of the matched facility characteristic items is reduced to execute the narrow-down again such that the number of data increases. On the other hand, in the second embodiment, by setting the priorities to the items instead of reducing "the number of items", "the facility characteristic item having the lowest priority" is excluded to execute the narrow-down again. The priorities can be set from the automatic analyzer 100. Fig. 10 is a diagram illustrating a configuration example of a narrow-down condition setting screen according to a second embodiment. On the narrow-down condition setting screen illustrated in Fig. 10, a field where the priority of each of the facility characteristic items can be set is added to the setting screen of Fig. 2. The priority can be set by anybody.

For example, as the priorities of the facility characteristic items in the present embodiment, the facility is set on the first priority, the sea level altitude is set on the next priority, the usage time period is set on the next priority, the number of days of use in one week is set on the next priority, the number of years of use of the device is set on the next priority, the manufacturer maintenance contract status is set on the next priority, the acquisition status of the ISO certification such as ISO 15189 is set on the next priority, the model is set on the next priority. The priorities can be changed at any time from the automatic analyzer 100. In the support center (server device) 200, when the number of the matched facility characteristics is narrowed down to the above-described 8 items in total and is insufficient for the required number of data, the facility characteristic item of the model that is set to the lowest priority is excluded, and the narrow-down process is executed again using the 7 items other than the model. When the number of data is less than the number of data required for the rearrangement as a result of executing the narrow-down again, in the support center (server device) 200, the acquisition status of the ISO certification such as ISO 15189 having the lowest priority is further excluded from the remaining seven facility characteristic items, and the narrow-down process is executed again using the 6 items other than the model and the acquisition status of the ISO certification such as ISO 15189. By repeating these processes, the narrow-down process is executed using the search range at a stage where the number of data is more than or equal to the number of data required for the narrow-down in the search range.

### (ii) Regarding Narrow-Down Process in Search Range

Even in the second embodiment, as in the first embodiment, the narrow-down using the number of recent data or the narrow-down using the acquisition period can be executed in the search range. For example, when information in one period is acquired, a method of setting an acquisition start date and an acquisition end date of the collection period or a method of narrowing down the alarm countermeasure information to information in March to May corresponding 3 years can be considered. For example, when the alarm countermeasure information in March to May corresponding 3 years is input, the input fields are considered to be as follows. Examples of the input fields include the acquisition start date (select a month and a date), the acquisition end date (select a month and a date), and the number of years (select A.D. and select the information corresponding to recent years). By executing the narrow-down in this period, for example, the tendency of an alarm that is likely to be generated in a cold period and a hot period can be reflected. Since the subsequent processes are the same as those of the first embodiment, the description thereof will not be repeated.

### (3) Conclusion

(i) The support center (server device) 200 according to the present embodiment executes: a process of narrowing down alarm countermeasure reference information (including information regarding various alarms and information regarding alarm countermeasures corresponding to the various alarms that are collected from a plurality of automatic analyzers) based on facility characteristic information including information regarding a use mode of a specific automatic analyzer that is acquired from the specific automatic analyzer and acquiring information regarding alarm countermeasures suitable for an alarm that is generated from the specific automatic analyzer; a process of rearranging the information regarding the alarm countermeasure obtained by the process based on a predetermined criterion; and a process of providing the rearranged information regarding the alarm countermeasure to the specific automatic analyzer via a communication line. Here, the alarm countermeasure reference information is associated with facility characteristic information of the plurality of automatic analyzers as information collection destinations. Therefore, the server device can narrow down information regarding an alarm countermeasure that is associated with the same facility characteristic information as those of the specific automatic analyzer (device as the alarm analysis target). In addition, the computer uses, as the predetermined criterion, information including at least one of the number of use cases of each of the alarm countermeasures or a maintenance status of the specific automatic analyzer. This way, when an alarm is generated in the automatic analyzer, by executing the narrow-down using the facility characteristics, the rearrangement of the alarm countermeasures on which the tendency in a similar facility is reflected can be executed. In addition, in the present embodiment, the narrow-down in each of the search periods (that can be set as the narrow-down condition in each of the automatic analyzers) can be executed. As a result, the rearrangement of the alarm countermeasures on which the trend in the period where the narrow-down is executed is reflected can be executed. With the above-described configuration, even the operator having little alarm correspondence experience can select an alarm countermeasure having higher effectiveness, and the alarm correspondence time can be reduced. The information regarding the number of use cases or the information regarding the maintenance status can be used by scoring each of the alarm countermeasures. For example, the information regarding the alarm countermeasures can be rearranged in order from an alarm countermeasure having the most use cases. In addition, a numerical value determined depending on a delay of the maintenance status of the specific automatic analyzer (the score can change depending on an elapsed time of the recommend maintenance period: the score is set such that the effectiveness of the related countermeasure increases as the elapsed time increases) is set to a score of the alarm countermeasure related to the maintenance status, and the information regarding the alarm countermeasures is rearranged based on the score of the alarm countermeasure. By adding up the score by the number of use cases and the score by the maintenance status, the effectiveness of each of the alarm countermeasures may be finally determined (rearranged).
(ii) When the number of the information regarding the alarm countermeasures extracted by the server device (support center 200) as a result of executing the narrow-down process does not reach a predetermined number, items of the facility characteristic information may be reduced to execute narrow-down process again such that the reliability of the alarm analysis result is ensured. At this time, the priority may be set to each of the items of the facility characteristic information such that the items of the facility characteristic information are reduced based on the priority. By setting the priority, an element that is regarded as important by the user can be reflected on the alarm countermeasure extraction result in the support center (server device) 200.
(iii) In the specific automatic analyzer where an alarm is generated, when the alarm is released (a malfunction/abnormality is corrected) by executing the alarm countermeasure presented from the support center 200, or when the alarm is released by an alarm countermeasure other than the alarm countermeasure presented from the support center 200, the information regarding the alarm countermeasure contributing to the release of the alarm may be added from the specific automatic analyzer to the alarm countermeasure reference information stored in a storage device (database) of the support center. As a result, the accuracy of the alarm analysis result in the support center 200 can be improved. In addition, the support center 200 can receive the information regarding the maintenance execution date from the plurality of automatic analyzers connected thereto, and can also add the received information to the alarm countermeasure reference information. By executing not only the rearrangement of the countermeasures using the number of use cases and but also the rearrangement of the countermeasures on which the maintenance status is reflected, even the operator having little alarm correspondence experience can execute alarm correspondence more rapidly. Regarding the collection of the alarm countermeasure information, the more specific alarm countermeasure information can be notified by collecting each of the portion of generation, the cause of generation, and the alarm countermeasure against the alarm to be corresponded instead of collecting whether or not the alarm can be released using the alarm countermeasure that is generated in advance.
(iv) The technique of the present disclosure can also be implemented by a program code of software. In this case, a storage medium that records the program code is provided to a system or a device, and a computer (or a CPU or an MPU) in the system or the device reads the program code stored in the storage medium. In this case, the program code itself read from the storage medium implements the functions of the above-described embodiments, and the program code itself and the storage medium recording the program code configure the present disclosure. As the storage medium for supplying the program code, for example, a flexible disk, a CD-ROM, a DVD-ROM, a hard disk, an optical disk, a magnetooptical disk, a CD-R, a magnetic tape, a non-volatile memory card, or a ROM is used.

In addition, an OS (operating system) or the like that operates on the computer may execute some or all of the actual processes based on an instruction of the program code to implement the functions of the above-described embodiments through the processes. Further, after writing the program code read from the storage medium into a memory in the computer, a CPU or the like on the computer may execute some or all of the actual processes based on an instruction of the program code to implement the functions of the above-described embodiments through the processes.

Further, the program code of the software implementing the functions of the embodiments may be distributed via a network such that the program code is stored in storage means such as a hard disk or a memory of a system or a device or in a storage medium such as a CD-RW or a CD-R, and a computer (or a CPU or an MPU) in the system or the device reads and executes the program code stored in the storage means or the storage medium during use.

The process and the technique described herein do not essentially relate to any specific device, and can also be implemented by any combination of suitable components. In order to execute the technique disclosed in the embodiments, a dedicated device may also be constructed. In addition, various aspects can be formed by an appropriate combination of the plurality of components disclosed in the embodiments. For example, some components may be removed from all the components disclosed in the embodiments, or components of the different embodiments may be appropriately combined.

Specific examples of the present disclosure have been described but do not limit the technique of the present disclosure. It can be understood by those skilled in the art that the present disclosure can be implemented by multiple combinations of suitable hardware, software, and firmware.

Further, in the above-described embodiments, the drawings illustrate control lines and information lines as considered necessary for explanations but do not illustrate all control lines or information lines in the products. All the configurations may be interconnected.

Further, it is obvious that those having common knowledge in the present technical field can implement other embodiments of the present disclosure in consideration of the contents of the present specification and the embodiments. The aspects and/or the components of the embodiments can be used alone or in any combination. The specification and the specific examples are merely typical examples, and the scope and concepts of the present disclosure are indicated by the following claims.

### Reference Signs List

- 100:: automatic analyzer
- 200:: support center (server device)
- 201, 207:: data storage unit
- 202, 208:: processing unit
- 203:: display unit
- 204:: input unit
- 205:: analysis unit
- 206, 209:: communication unit

## Claims

1. A data analysis method in which information regarding countermeasures of an alarm generated from a specific automatic analyzer is provided to the specific automatic analyzer using a computer, the data analysis method comprising:
collecting alarm countermeasure reference information including information regarding various alarms and information regarding alarm countermeasures corresponding to the various alarms from a plurality of automatic analyzers; and
analyzing the information regarding the alarm generated from the specific automatic analyzer, extracting an alarm countermeasure that is effective for releasing the alarm from the alarm countermeasure reference information, and transmitting the extracted alarm countermeasure to the specific automatic analyzer,
wherein the extraction of the alarm countermeasure of the alarm countermeasure reference information and the transmission of the extracted alarm countermeasure to the specific automatic analyzer includes
narrowing down the alarm countermeasure reference information based on facility characteristic information including information regarding a use mode of the specific automatic analyzer that is acquired from the specific automatic analyzer and acquiring information regarding countermeasures suitable for the alarm that is generated from the specific automatic analyzer,
rearranging the acquired information regarding the countermeasures suitable for the alarm based on a predetermined criterion, and
providing the rearranged information regarding the countermeasures suitable for the alarm to the specific automatic analyzer via a communication line.

2. The data analysis method according to claim 1,
wherein the alarm countermeasure reference information is associated with facility characteristic information of the plurality of automatic analyzers as information collection destinations, and
the computer narrows down the information regarding the countermeasures suitable for the alarm associated with the same facility characteristic information as that of the specific automatic analyzer from which the alarm is generated.

3. The data analysis method according to claim 1,
wherein the computer uses, as the predetermined criterion, information including at least one of the number of use cases of each of the countermeasures suitable for the alarm or a maintenance status of the specific automatic analyzer, and rearranges the information regarding the countermeasures suitable for the alarm based on the criterion.

4. The data analysis method according to claim 3,
wherein the computer rearranges the information regarding alarm countermeasures in order from an alarm countermeasure having the most use cases.

5. The data analysis method according to claim 3,
wherein the computer sets a numerical value determined depending on a delay of the maintenance status of the specific automatic analyzer to a score of the alarm countermeasure related to the maintenance status, and rearranges the information regarding the countermeasures suitable for the alarm based on the score of the alarm countermeasure.

6. The data analysis method according to claim 3,
wherein the computer sets the number of use cases of each of the alarm countermeasures to a first type score of the corresponding alarm countermeasure, sets a numerical value determined depending on a delay of the maintenance status of the specific automatic analyzer to a second type score of the alarm countermeasure related to the maintenance status, and rearranges the information regarding the countermeasures suitable for the alarm based on a total score of the alarm countermeasure obtained by adding up the first type score and the second type score.

7. The data analysis method according to claim 1,
wherein the facility characteristic information includes a plurality of items regarding a use mode of the automatic analyzer, and
when the number of the information regarding the countermeasures suitable for the alarm does not reach a predetermined number, the computer reduces the items of the facility characteristic information to acquire the information regarding the countermeasures suitable for the alarm again.

8. The data analysis method according to claim 7,
wherein priorities are set to the plurality of items of the facility characteristic information, and
the computer reduces the items of the facility characteristic information based on the priorities.

9. The data analysis method according to claim 1,
wherein the facility characteristic information includes a plurality of pieces of information among information regarding a facility where the automatic analyzer is provided, information regarding an altitude of a location where the automatic analyzer is provided, information regarding a usage time period of the automatic analyzer in one day, information regarding the number of days of use of the automatic analyzer in one week, information regarding the number of years of use of the automatic analyzer, information regarding a manufacturer maintenance contract of the automatic analyzer, information regarding an acquisition status of an ISO certification, and information regarding a model of the automatic analyzer.

10. The data analysis method according to claim 1,
wherein the computer associates information regarding an alarm countermeasure contributing to release of the alarm that is transmitted from the specific automatic analyzer with the information regarding the alarm and the facility characteristic information of the specific automatic analyzer, and adds the associated information to the information regarding the countermeasures of the alarm.

11. The data analysis method according to claim 1,
wherein the computer adds information regarding a maintenance execution date of the corresponding automatic analyzer that is transmitted from the plurality of automatic analyzers to the alarm countermeasure reference information.

12. The data analysis method according to claim 1, further comprising:
allowing the specific automatic analyzer to receive rearranged information regarding the countermeasures suitable for the alarm; and
allowing the specific automatic analyzer to display the information regarding the countermeasures suitable for the alarm on a display screen to urge to execute the countermeasures suitable for the alarm in a rearranged order.

13. A server device that provides information regarding countermeasures of an alarm generated from a specific automatic analyzer to the specific automatic analyzer, the server device comprising:
a processor configured to analyze alarm information acquired from a plurality of automatic analyzers; and
a communication device configured to transmit the information regarding the countermeasures of the alarm to the specific automatic analyzer,
wherein the processor executes
a process of acquiring reference data including information regarding at least one of the plurality of automatic analyzers, information regarding a reagent, information regarding a usage environment and a usage form of a facility where at least one automatic analyzer is provided, and the alarm information corresponding to the above information from the at least automatic analyzer, and
a process of rearranging the information regarding the countermeasures of the alarm for the automatic analyzer in an execution priority order based on the reference data.

14. The server device according to claim 13,
wherein the processor further executes a process of allowing the communication device to transmit the information regarding the countermeasures of the alarm that is rearranged in the execution priority order to the specific automatic analyzer as an analysis target.
